# FASCICULE DE BREVET EUROPEEN

(11) **EP 3 773 432 B1**
(45) Date de publication et mention de la délivrance du brevet: **10.09.2025**
(21) Numéro de dépôt: 19715534.4
(22) Date de dépôt: 10.04.2019
(51) Int. Cl.: A61K 8/31, A61Q 1/02, A61Q 1/14, A61Q 19/00

(54) **EXCIPIENT COSMETIQUE COMPRENANT UN ALCANE EN C8-C10 ET UN ALCANE EN C>=11**
KOSMETISCHER TRÄGERSTOFF MIT EINEM ALKAN MIT C8-C10 UND EINEM ALKAN MIT C>=11
COSMETIC EXCIPIENT COMPRISING A C8-C10 ALKANE AND A C>=11 ALKANE

(30) Priorité: 10.04.2018 FR 1853142
(43) Date de publication de la demande: 17.02.2021
(60) Demande divisionnaire: 22214400.8
(73) Titulaire: Biosynthis, 91410 Saint Cyr Sous Dourdan (FR)
(72) Inventeur: BERNOUD, Thierry, 91410 SAINT CYR SOUS DOURDAN (FR)
(74) Mandataire: Tripoz, Inès
(86) Numéro de dépôt international: PCT/EP2019/059172
(87) Numéro de publication internationale: WO 2019/197507

(56) Documents cités:
- FR-A1- 2 952 528
- US-A- 4 849 211
- US-A1- 2015 190 321
- NIKITAKIS JOANNE ET AL: "International Cosmetic Ingredient Dictionary and Handbook Sixteenth Edition 2016 Volume 1 Editorial Advisory Board", 1 January 2016 (2016-01-01), pages 1 - 61, XP055796044, Retrieved from the Internet <URL:https://webdictionary.personalcarecouncil.org/ctfa-static/online/FrontMatter_Vol1%20Edited%20for%20Websites.pdf> [retrieved on 20210416]
- MATTHEWS M.: "Diffusion Coefficients for Binary Alkane Mixtures to 573 K and 3.5 MPa", AICHE JOURNAL, vol. 33, no. 6, 1 January 1987 (1987-01-01), XP055861771
- 1 January 2016, INTERNATIONAL COSMETIC INGREDIENT DICTIONARY AND HANDBOOK, PERSONAL CARE PRODUCTS COUNCIL- WINCI, ISBN: 978-1-882621-55-2, article ANONYME: "Coconut Alkanes: Vegelight 1214LC (pg 855) and Gransil GVL (pg 6281)", pages: 855, 6281, XP002786808
- ANONYME: "SPECIALITIES Tab. 2: Zusammensetzung und Eigenschaften natürlicher Öle und Fette", SÖFW-JOURNAL, vol. 127, no. 7, July 2001 (2001-07-01), pages 90, XP002787778
- ANONYME: "Soltrol Isoparaffin Solvents", 21 December 2000 (2000-12-21), XP002786809, Retrieved from the Internet <URL:http://www.cpchem.com/bl/specchem/en-us/tdslibrary/CPChem%20Soltrol%20Brochure.pdf>
- DATABASE ILRC Database [online] National Center for Forensic Science, University of Central Florida; 9 June 2006 (2006-06-09), "Phillips 66 Soltrol 130", XP002787779, Database accession no. 0412

## Description

L'invention concerne le domaine technique des soins cosmétiques de la peau et des phanères.

Plus précisément, l'invention concerne un excipient cosmétique, ainsi qu'une formulation cosmétique.

Un certain nombre de définitions doivent être données.

On appelle « alcane » un hydrocarbure saturé étant constitué uniquement d'atomes de carbone et d'hydrogène liés entre eux par des liaisons covalentes simples dont la formule générale est CₙH₂ₙ₊₂.

On appelle « alcane linéaire » un alcane dans lequel chaque atome de carbone est lié au maximum à deux atomes de carbone.

On appelle « alcane ramifié » un alcane dans lequel certains atomes de carbone sont liés à trois, voire quatre atomes de carbone.

On appelle « alcane en Cₓ » un alcane constitué de x atomes de carbone, ledit alcane pouvant être linéaire ou ramifié. Par exemple, le n-décane est un alcane en C10.

On appelle « alcane en C ≤ x » un alcane constitué d'un nombre de carbone de x ou moins de x. Par exemple, un alcane en C ≤ 2 peut être en C1 ou en C2.

On appelle « alcane en C ≥ x » un alcane constitué d'un nombre de carbone de x ou plus de x. Par exemple, un alcane en C ≥ 2 peut être tout alcane, à l'exclusion du méthane (C1).

On appelle « alcane en Cₓ-C_{y} » un alcane constitué de x à y atomes de carbone, ledit alcane pouvant être linéaire ou ramifié. Par exemple, les alcanes en C1-C2 sont le méthane et l'éthane.

On appelle « méthylalcane » un alcane ramifié comprenant au moins une ramification étant un groupement méthyl. Par exemple, le 4-méthylnonane, ou encore le 2,3-diméthyloctane, sont des méthylalcanes, et plus précisément des méthylalcanes en C10. Par exemple les méthylundécanes sont des méthylalcanes en C12. Par exemples les diméthyloctanes sont méthylalcanes en C12. Les méthyleheptadécanes sont des alcanes en C18. Ces méthylalcanes sont préférentiellement obtenus par déshydratation d'un alcool de Guerbet. Lorsque des carbones asymétriques sont présents, on entend par alcanes ramifiés, les mélanges racémiques et les isomères séparés.

On appelle « alcane désodorisé » un alcane ayant subi une opération de désodorisation, ladite désodorisation permettant d'éliminer les impuretés responsables d'une odeur désagréable pour l'utilisateur. Ces impuretés peuvent être notamment des résidus des alcools de Guerbet qui sont les produits de départ le plus couramment utilisés. Ces taux très faibles sont obtenus grâce à un rendement de déshydratation très élevé. Un alcane désodorisé selon l'invention est un alcane qui comprend moins de 0,01 % de résidu d'alcool de Guerbet correspondant.

Un alcane déodorisé est par ailleurs exempt de composés soufrés comme les thiols, par exemple le 1-nonanethiol ; on entend par exempt une teneur inférieure à 0,01 %.

On appelle « point éclair » le point éclair mesuré selon la norme ASTM D93. L'unité du point éclair mesuré selon la norme ASTM D93 est le degré Celsius (°C). Il peut également s'agir d'une moyenne de valeurs, chacune des valeurs étant obtenue au moyen d'une mesure selon la norme ASTM D93.

On appelle « pourcentage d'évaporation à t=X » le pourcentage de composé (ou de mélange de composés) évaporés à un temps donné (X), les conditions d'évaporation étant les suivantes : dessiccateur LABOMODERNE THERMOBALANCE KERN DBS 60-3, 20°C, sous atmosphère contrôlée. Par exemple, si le pourcentage d'évaporation est d'au moins 60% après 3 heures, cela signifie qu'au moins 60% du composé (ou du mélange de composés) s'est évaporé après 3 heures.

On appelle « pourcentage massique » le rapport de la masse d'un premier composé par rapport à la masse totale d'un mélange de composés (comprenant le premier composé) ou composition, ramené à un pourcentage. Par exemple, si 10 grammes d'un composé sont présents dans un mélange z ayant une masse totale de 100 grammes, alors le pourcentage massique de y dans z est 10%.

On « appelle pourcentage en poids », le rapport du poids d'un constituant par rapport au poids total de la composition, ramené à 100.

Dans le cadre de la présente demande, le terme « environ » précédent une valeur numérique signifie que la valeur peut être modifiée de +/- 10%. Dans le cas particulier d'une valeur numérique étant une borne d'intervalle, le terme « environ » signifie que la borne basse peut être réduite de 10% ou la borne haute peut être augmentée de 10%. Il est également possible de supprimer le terme « environ » précédent une valeur numérique.

Depuis l'apparition des silicones en cosmétique dans les années 1950, leur utilisation s'est considérablement étendue dans tous les domaines de la cosmétique, des produits pour le soin de la peau aux produits de maquillage en passant par les produits capillaires. Cette large utilisation s'explique par les caractéristiques physico-chimiques spécifiques des silicones. Il existe 5 grandes catégories de silicones : les huiles de silicones volatiles, les huiles de silicones non volatiles, les huiles de silicones modifiées, les cires de silicones, ainsi que les gommes de silicone.

Les huiles de silicones volatiles sont bien souvent composées de silicones cycliques à chaînes courtes de formule générale [Si-(CH₃)₂-O]ₙ avec n étant compris entre 3 et 7 (bornes incluses). Elles sont généralement appelées cyclométhicones, et ceci quel que soit le nombre de motifs. Les cyclométhicones les plus communes sont le cyclotétrasiloxane (D4) (C₈H₂₄O₄Si₄, CAS 556-67-2), le cyclopentasiloxane (D5) (C₁₀H₃₀O₅Si₅, CAS 541-02-6) et le cyclohexasiloxane (D6) (C₁₂H₃₆O₆Si₆, CAS 540-97-6).

Les cyclométhicones ont, jusqu'à une époque très récente, été considérées comme des émollients et solvants inoffensifs pour la peau (voir par exemple International Journal of Toxicology, Volume 10, n°1, pp. 9-19 1991*)* ; ce n'est que relativement récemment que leurs potentiels effets délétères sur l'environnement, voire sur la santé humaine, ont été mis en évidence.

Des recherches ont dès lors été réalisées afin d'identifier des composés susceptibles d'offrir une alternative satisfaisante, ces composés devant posséder des caractéristiques de volatilité proches et un comportement similaire dans une formulation, en particulier sur le plan de la viscosité, des propriétés sensorielles (capacité d'étalement sur la peau et de toucher doux, « sec » et non gras du film obtenu).

Les propriétés sensorielles sont testées par des panels, appelés panels sensoriels voir par exemple Concepton des produits cosmétiques : la formulation, Anne-Marie PENSE-LHERITIER, page 95, LAVOISIER.

Il a par exemple été proposé dans la demande US20050079986 au nom de COGNIS une composition huileuse renfermant un mélange d'un dialkyl carbonate linéaire ou ramifié avec un alcane, de préférence ramifié et saturé, contenant de 8 à 40 atomes de carbone.

Il a également été suggéré dans la demande US20040241200 au nom de INOLEX d'utiliser une association d'isoparaffine telle que l'isododécane avec un polyester de néopentylglycol, tel que le diheptanoate de néopentylglycol. Ce mélange est notamment commercialisé par la société INOLEX sous la dénomination commerciale LexFeel^{®} D4 et D5. La société PRESPERSE LLC propose également, sous la dénomination commerciale SiClone^{®} SR-5, un mélange d'isoparaffines en C₁₃-C₁₆, C₁₂-C₁₄ avec des alcanes en C₁₃-C₁₅.

En outre, il a été proposé dans le brevet US6126951 au nom de BERNEL l'utilisation d'un isostéarate de caprylyle résultant de l'estérification du 2-octanol par un mélange d'isomères d'acides gras en C₁₈, sans toutefois atteindre la volatilité recherchée.

Enfin, dans la demande WO2010115973 au nom de la Demanderesse, il a été proposé l'utilisation d'un mélange d'alcanes. FR 2 952 528 A1 (OREAL [FR]) propose un mélange d'alcanes comprenant au moins du n-dodécane et du n-tétradécane. Dans une longue liste d'additifs possibles est cité l'isodécane.

Il subsiste toutefois le besoin de disposer d'un excipient cosmétique présentant des caractéristiques physico-chimiques et sensorielles encore plus proches de celles des cyclométhicones et susceptibles de s'y substituer, notamment en tant qu'excipient à inclure dans des formulations cosmétiques.

De manière surprenante, il a été mis en évidence par la Demanderesse qu'un excipient cosmétique idéal eu égard aux objectifs identifiés précédemment pouvait être obtenu.

De manière surprenante, il a été mis en évidence qu'au sein d'un mélange d'alcanes, les alcanes en C8-C10 (et plus particulièrement le C10) ont une influence particulière et non prédictible sur les propriétés de volatilité du mélange d'alcanes (notamment appréciées par la mesure de l'évaporation et du point éclair).

Par exemple, un mélange d'alcane comprenant 30% de n-decane (C10, point éclair 46°C), 40% de n-dodecane (C12, point éclair 71°C) et 30% de n-tetradecane (C14, point éclair 115°C) a un point éclair de 66°C, ce qui n'était pas prévisible.

L'excipient cosmétique selon l'invention comprend un mélange d'alcanes, ledit mélange ayant un point éclair inférieur à 69°C, de préférence compris entre 60 et 69°C, de préférence compris entre 60 et 67°C, de préférence compris entre 62 et 65°C.

Il a également été mise en évidence que l'utilisation d'un excipient cosmétique constitué uniquement d'alcanes ramifiés, permettait d'obtenir des émulsions plus stables que les émulsions obtenues avec des alcanes linéaires.

Il a également été mis en évidence que l'inclusion d'un excipient cosmétique selon l'invention dans des formulations cosmétiques avait pour effet de procurer à ladite formulation cosmétique de remarquables qualités cosmétiques outre une excellente stabilité des émulsions comme une meilleure dispersibilité, à savoir la possibilité d'obtenir des dispersions régulières, des poudres par exemple les pigments et un meilleur étalement des compositions sur la peau, ces propriétés étant particulièrement importantes pour les produits de maquillage comme les fonds de teint et les produits solaires.

L'invention concerne un excipient cosmétique comprenant un mélange d'alcanes, ledit mélange d'alcanes comprenant au moins un alcane en C8-C10, et au moins un alcane en C ≥ 11, selon la revendication 1.

Dans un mode de réalisation, ledit mélange d'alcanes est un mélange d'alcanes dont le nombre d'atomes est pair.

Ledit mélange d'alcanes est exempt d'alcanes dont le nombre de carbone est impair.

Cette parité est due à l'origine naturelle des alcools qui sont utilisés comme produits de départ que ce soit pour la synthèse des alcools de Guerbet ou qui sont déshydratés pour obtenir lesdits alcanes, en effet les alcools naturels comportent un nombre pair d'atomes de carbone.

L'excipient cosmétique selon l'invention, est caractérisé en ce que ledit au moins un alcane en C8-C10 est au moins un alcane en C10.

Dans un mode de réalisation, l'excipient cosmétique selon l'invention, est caractérisé en ce que ledit au moins un alcane en C8-C10 est au moins un alcane linéaire ou ramifié en C10.

Dans un mode de réalisation, l'excipient cosmétique selon l'invention, est caractérisé en ce que ledit au moins un alcane en C8-C10 est au moins un alcane linéaire en C10.

Dans un mode de réalisation, l'excipient cosmétique selon l'invention, est caractérisé en ce que ledit au moins un alcane en C8-C10 est au moins un alcane ramifié en C10.

Dans un mode de réalisation, l'excipient cosmétique selon l'invention est caractérisé en ce que ledit au moins un alcane en C8-C10 est au moins un alcane en C10 ramifié choisi dans le groupe constitué des méthylnonanes, comme par exemple le 2-méthylnonane (CAS 871-83-0), des éthyloctanes, comme par exemple le 3-éthyloctane (CAS 5881-17-4), des diméthyloctanes, comme par exemple le 2,2-diméthyloctane (CAS 15869-87-1), des propylheptanes, comme par exemple le 4-propylheptane (CAS 3178-29-8), des éthylméthylheptanes, comme par exemple le 3-éthyl-2-méthylheptane (CAS 14676-29-0), des triméthylheptanes, comme par exemple le 2,2,3-triméthylheptane (CAS 52896-92-1), des diéthylhexanes, comme par exemple le 3,3-diéthylhexane (CAS 17302-02-2), des tétraméthylhexanes, comme par exemple le 2,2,3,3-tétraméthylhexane (CAS 13475-81-5), des éthyltriméthylpentanes, comme par exemple le 3-éthyl-2,2,3-triméthylpentane (CAS 52897-17-3), et leurs mélanges.

Dans un mode de réalisation, l'excipient cosmétique selon l'invention, est caractérisé en ce que ledit au moins un alcane en C8-C10 est au moins un méthyl alcane en C10.

Dans un mode de réalisation, l'excipient cosmétique selon l'invention, est caractérisé en ce que ledit au moins un alcane en C8-C10 est choisi dans le groupe constitué du 4-methylnonane (C10H22, CAS 17301-94-9), du 2-methylnonane (C10H22, CAS 871-83-0), du n-decane (C10H22, CAS 124-18-5), et de leurs mélanges.

Dans un mode de réalisation, l'excipient cosmétique selon l'invention, est caractérisé en ce que ledit au moins un alcane en C8-C10 est au moins le 4-methylnonane.

Dans un mode de réalisation, l'excipient cosmétique selon l'invention, est caractérisé en ce que ledit au moins un alcane en C8-C10 est au moins le 2-methylnonane.

Dans un mode de réalisation, l'excipient cosmétique selon l'invention, est caractérisé en ce que ledit au moins un alcane en C8-C10 est au moins le n-décane. Le n-décane est particulièrement préféré.

L'excipient cosmétique selon l'invention, est caractérisé en ce que ledit au moins un alcane en C8-C10 est présent en un pourcentage massique compris entre 10 et 30% (10% ≤ pourcentage massique ≤ 30%), par rapport à la masse totale dudit mélange d'alcanes.

Dans un mode de réalisation, l'excipient cosmétique selon l'invention, est caractérisé en ce que ledit au moins un alcane en C8-C10 est présent en un pourcentage massique compris entre environ 15 et environ 30% (15% ≤ pourcentage massique ≤ 30%), par rapport à la masse totale dudit mélange d'alcanes.

Dans un mode de réalisation, l'excipient cosmétique selon l'invention, est caractérisé en ce que ledit au moins un alcane en C ≥ 11 est au moins un alcane linéaire en C12.

Dans un mode de réalisation, l'excipient cosmétique selon l'invention, est caractérisé en ce que ledit au moins un alcane en C ≥ 11 est au moins le n-dodecane.

L'invention concerne également un excipient cosmétique comprenant un mélange d'alcanes tel que précédemment défini, caractérisé en ce que ledit au moins un alcane en C ≥ 11 est présent en un pourcentage massique compris entre environ 50 et environ 99% (50% ≤ pourcentage massique ≤ 99%), par rapport à la masse totale dudit mélange d'alcanes.

L'invention concerne également un excipient cosmétique comprenant un mélange d'alcanes tel que précédemment défini, caractérisé en ce que ledit au moins un alcane en C ≥ 11 est présent en un pourcentage massique compris entre environ 60 et environ 99% (50% ≤ pourcentage massique ≤ 99%), par rapport à la masse totale dudit mélange d'alcanes.Dans un mode de réalisation, l'excipient cosmétique selon l'invention, est caractérisé en ce que ledit au moins un alcane en C ≥ 11 est présent en un pourcentage massique compris entre environ 70 et environ 99% (70% ≤ pourcentage massique ≤ 99%), par rapport à la masse totale dudit mélange d'alcanes.

Dans un mode de réalisation, l'excipient cosmétique selon l'invention, est caractérisé en ce que ledit au moins un alcane en C ≥ 11 est présent en un pourcentage massique compris entre environ 80 et environ 99% (70% ≤ pourcentage massique ≤ 99%), par rapport à la masse totale dudit mélange d'alcanes

Dans un mode de réalisation, l'excipient cosmétique selon l'invention, est caractérisé en ce que ledit au moins un alcane en C ≥ 11 est présent en un pourcentage massique compris entre environ 70 et environ 95% (70% ≤ pourcentage massique ≤ 95%), par rapport à la masse totale dudit mélange d'alcanes.

Dans un mode de réalisation, l'excipient cosmétique selon l'invention, est caractérisé en ce que ledit au moins un alcane en C ≥ 11 est présent en un pourcentage massique compris entre environ 70 et environ 90% (70% ≤ pourcentage massique ≤ 90%), par rapport à la masse totale dudit mélange d'alcanes.

Dans un mode de réalisation, l'excipient cosmétique selon l'invention, est caractérisé en ce que ledit au moins un alcane en C ≥ 11 est présent en un pourcentage massique compris entre environ 70 et environ 85% (70% ≤ pourcentage massique ≤ 85%), par rapport à la masse totale dudit mélange d'alcanes.

L'excipient cosmétique selon l'invention, est caractérisé en ce que ledit au moins un alcane en C8-C10 est un alcane en C10, et l'au moins un alcane en C ≥ 11 est un alcane linéaire en C12.

L'invention concerne également un excipient cosmétique comprenant un mélange d'alcanes tel que précédemment défini, caractérisé en ce que ledit au moins un alcane en C8-C10 est au moins le n-décane présent dans ledit excipient à un pourcentage massique compris entre environ 15 et environ 25% (15% ≤ pourcentage massique ≤ 25%) par rapport à la masse totale dudit mélange d'alcane, et l'au moins un alcane en C ≥ 11 est un mélange de n-dodecane présent dans ledit excipient à un pourcentage massique compris entre environ 20 et environ 60% (20% ≤ pourcentage massique ≤ 60%) par rapport à la masse totale dudit mélange d'alcane et de n-tetradecane présent dans ledit excipient à un pourcentage massique compris entre environ 15 et environ 45% (15% ≤ pourcentage massique ≤ 45%) par rapport à la masse totale dudit mélange d'alcane.

L'invention concerne également un excipient cosmétique comprenant un mélange d'alcanes tel que précédemment défini, caractérisé en ce que ledit au moins un alcane en C8-C10 est au moins le n-décane présent dans ledit excipient à un pourcentage massique d'environ 30% par rapport à la masse totale dudit mélange d'alcane, et l'au moins un alcane en C ≥ 11 est un mélange de n-dodecane présent dans ledit excipient à un pourcentage massique d'environ 40% par rapport à la masse totale dudit mélange d'alcane et de n-tetradecane présent dans ledit excipient à un pourcentage massique d'environ 30% par rapport à la masse totale dudit mélange d'alcane.

L'invention concerne également un excipient cosmétique comprenant un mélange d'alcanes tel que précédemment défini, caractérisé en ce que ledit au moins un alcane en C8-C10 est au moins le n-décane présent dans ledit excipient à un pourcentage massique d'environ 20% par rapport à la masse totale dudit mélange d'alcane, et l'au moins un alcane en C ≥ 11 est un mélange de n-dodecane présent dans ledit excipient à un pourcentage massique d'environ 74% par rapport à la masse totale dudit mélange d'alcane et de n-tetradecane présent dans ledit excipient à un pourcentage massique d'environ 6% par rapport à la masse totale dudit mélange d'alcane.

Dans un mode de réalisation, ledit mélange d'alcane comprend :
- 30% de n-decane ;
- 40% de n-dodecane ; et
- 30% de n-tetradecane.

Dans un mode de réalisation, ledit mélange d'alcane comprend :
- 30% de n-decane ;
- 40% de n-dodecane ; et
- 30% de n-tetradecane.

Dans un mode de réalisation, ledit mélange d'alcane comprend :
- 10% de n-décane ;
- 85% de n-dodécane ; et
- 5% de n-tétradécane.

Dans un mode de réalisation, ledit mélange d'alcane comprend :
- 15% de n-décane ;
- 80% de n-dodécane ; et
- 5% de n-tétradécane.

Dans un mode de réalisation, ledit mélange d'alcane comprend :
- 20% de n-décane ;
- 75,6% de n-dodécane ; et
- 4,4% de n-tétradécane.

Dans un mode de réalisation, ledit mélange d'alcane comprend :
- 20% de n-décane ;
- 74% de n-dodécane ; et
- 6% de n-tétradécane.

Dans un mode de réalisation, ledit mélange d'alcane comprend :
- 20% de n-décane ;
- 70% de n-dodécane ; et
- 10% de n-tétradécane.

Dans un mode de réalisation, ledit mélange d'alcane comprend :
- 20% de n-décane ;
- 75% de n-dodécane ; et
- 5% de n-tétradécane.

L'invention concerne également un excipient cosmétique comprenant un mélange d'alcanes tel que précédemment défini, caractérisé en ce qu'en outre un silicone non volatile comme par exemple le polysilicone 11.

L'invention concerne également un excipient cosmétique comprenant un mélange d'alcanes tel que précédemment défini, ledit excipient comprenant en outre du polysilicone 11, caractérisé en ce que ledit mélange d'alcanes est présent en un pourcentage massique compris entre environ 65 et environ 95% (65% ≤ pourcentage massique ≤ 95%) par rapport à la masse totale dudit excipient, et ledit polysilicone 11 est présent en un pourcentage massique compris entre environ 5 et environ 35% (5% ≤ pourcentage massique ≤ 35%) par rapport à la masse totale dudit excipient.

L'invention concerne également un excipient cosmétique comprenant un mélange d'alcanes tel que précédemment défini, ledit excipient comprenant en outre du polysilicone 11, caractérisé en ce que ledit mélange d'alcanes est présent en un pourcentage massique compris entre environ 75 et environ 85% (75% ≤ pourcentage massique ≤ 85%) par rapport à la masse totale dudit excipient, et ledit polysilicone 11 est présent en un pourcentage massique compris entre environ 15 et environ 25% (15% ≤ pourcentage massique ≤ 25%) par rapport à la masse totale dudit excipient.

L'invention concerne également un excipient cosmétique comprenant un mélange d'alcanes tel que précédemment défini, ledit excipient comprenant en outre du polysilicone 11, caractérisé en ce que ledit mélange d'alcanes est présent en un pourcentage massique compris entre environ 80 et environ 82% (80% ≤ pourcentage massique ≤ 82%) par rapport à la masse totale dudit excipient, et ledit polysilicone 11 est présent en un pourcentage massique compris entre environ 18 et environ 20% (18% ≤ pourcentage massique ≤ 20%) par rapport à la masse totale dudit excipient.

L'invention concerne également un excipient cosmétique comprenant un mélange d'alcanes tel que précédemment défini, caractérisé en ce que ledit mélange d'alcane a un point éclair inférieur ou égal à 69°C (≤ 69°C).

L'invention concerne également un excipient cosmétique comprenant un mélange d'alcanes tel que précédemment défini, caractérisé en ce que ledit mélange d'alcane a un point éclair compris 60 et 69°C (60°C ≤ point éclair ≤ 69°C).

L'invention concerne également un excipient cosmétique comprenant un mélange d'alcanes tel que précédemment défini, caractérisé en ce que ledit mélange d'alcane a un point éclair compris entre 60 et 67°C (60°C ≤ point éclair ≤ 67°C).

L'invention concerne également un excipient cosmétique comprenant un mélange d'alcanes tel que précédemment défini, caractérisé en ce que ledit mélange d'alcane a un point éclair compris entre 62 et 65°C (62°C ≤ point éclair ≤ 65°C).

L'invention concerne également un excipient cosmétique comprenant un mélange d'alcanes tel que précédemment défini, caractérisé en ce que le pourcentage d'évaporation à t=10 heures est compris entre 10 et 30 % (10 % ≤ pourcentage d'évaporation à t=10 h ≤ 30 %).

L'invention concerne également un excipient cosmétique comprenant un mélange d'alcanes tel que précédemment défini, caractérisé en ce que le pourcentage d'évaporation à t=10 heures est compris entre 12 et 20 % (12 % ≤ pourcentage d'évaporation à t=10 h ≤ 20 %).

Excipient cosmétique selon l'invention tel que précédemment défini, caractérisé en ce qu'il est intégralement obtenu à partir de matières premières d'origine végétale, bactérienne ou animale, de préférence d'origine végétale.

Dans un mode de réalisation, les alcanes selon l'invention sont obtenus par déshydratation de l'alcool de Guerbet correspondant, suivi d'une hydrogénation de l'alcène obtenu.

Par exemple, le 5-méthylundécane sera obtenu par déshydratation du 2-butyloctanol-1 selon le schéma réactionnel ci-dessous :

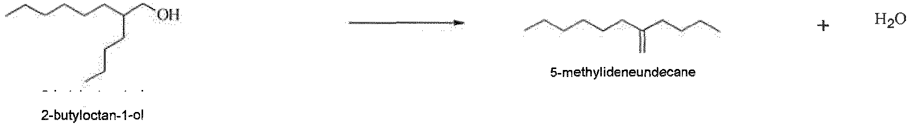

Puis hydrogénation de l'alcène obtenu selon le schéma réactionnel ci-dessous :

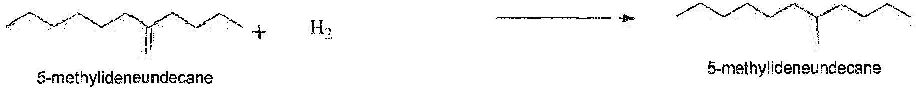

L'alcool de Guerbet sera obtenu soit par un procédé de Guerbet classique par condensation de deux alcools , soit par une synthèse telle que décrite dans la demande de brevet US2012/0220806.

utilisations suivantes sont divulguées mais non revendiquées:

Utilisation d'un excipient cosmétique selon l'invention tel que précédemment défini, dans la préparation d'une formulation cosmétique de type mascara

Utilisation d'un excipient cosmétique selon l'invention tel que précédemment défini, dans la préparation d'une formulation cosmétique démaquillante biphasique.

Utilisation d'un excipient cosmétique selon l'invention tel que précédemment défini, dans la préparation d'une formulation cosmétique pour la coloration des lèvres.

Utilisation d'un excipient cosmétique selon l'invention tel que précédemment défini, dans la préparation d'une formulation cosmétique de type capillaire rincée ou non rincée.

Utilisation d'un excipient cosmétique selon l'invention tel que précédemment défini, dans la préparation d'une formulation cosmétique étant une émulsion.

Utilisation d'un excipient cosmétique selon l'invention tel que précédemment défini, dans la préparation d'une phase huileuse.

Utilisation d'un excipient cosmétique selon l'invention tel que précédemment défini, dans la préparation d'une phase huileuse destinée à une émulsion.

Utilisation d'un excipient cosmétique selon l'invention tel que précédemment défini, en substitution d'au moins une huile de silicone volatile.

Utilisation d'un excipient cosmétique selon l'invention tel que précédemment défini, en substitution d'au moins une huile de silicone volatile choisie dans le groupe comprenant le cyclotétrasiloxane (D4) (C8H24O4Si4, CAS 556-67-2), le cyclopentasiloxane (D5) (C10H30O5Si5, CAS 541-02-6), le cyclohexasiloxane (D6) (C12H36O6Si6, CAS 540-97-6), et leurs mélanges.

Utilisation d'un excipient cosmétique selon l'invention tel que précédemment défini, en substitution d'au moins une huile de silicone volatile étant le cyclotétrasiloxane (D4).

Utilisation d'un excipient cosmétique selon l'invention tel que précédemment défini, en substitution d'au moins une huile de silicone volatile étant le cyclopentasiloxane (D5).

Utilisation d'un excipient cosmétique selon l'invention tel que précédemment défini, en substitution d'au moins une huile de silicone volatile étant le cyclohexasiloxane (D6).

Utilisation d'un excipient cosmétique selon l'invention tel que précédemment défini, en substitution d'au moins une huile de silicone volatile choisie dans le groupe comprenant le cyclotétrasiloxane (D4) (C8H24O4Si4, CAS 556-67-2), le cyclopentasiloxane (D5) (C10H30O5Si5, CAS 541-02-6), le cyclohexasiloxane (D6) (C12H36O6Si6, CAS 540-97-6), et leurs mélanges, caractérisée en ce que ladite substitution est effectuée en conservant la même quantité massique. Cela signifie qu'une certaine masse d'huile silicone est remplacée par une même masse d'excipient cosmétique selon l'invention.

Utilisation d'un excipient cosmétique selon l'invention tel que précédemment défini, en substitution d'au moins une huile de silicone volatile étant le cyclotétrasiloxane (D4), caractérisée en ce que ladite substitution est effectuée en conservant la même quantité massique.

Utilisation d'un excipient cosmétique selon l'invention tel que précédemment défini, en substitution d'au moins une huile de silicone volatile étant le cyclopentasiloxane (D5), caractérisée en ce que ladite substitution est effectuée en conservant la même quantité massique.

Utilisation d'un excipient cosmétique selon l'invention tel que précédemment défini, en substitution d'au moins une huile de silicone volatile étant le cyclohexasiloxane (D6), caractérisée en ce que ladite substitution est effectuée en conservant la même quantité massique.

Formulation cosmétique caractérisée en ce qu'elle comprend, en tant qu'excipient cosmétique, un excipient cosmétique selon l'invention tel que précédemment défini.

L'invention concerne également une formulation cosmétique caractérisée en ce qu'elle comprend un mélange d'alcanes, ledit mélange d'alcanes ayant un point éclair inférieur ou égal à 69°C (≤ 69°C).

Formulation cosmétique selon l'invention tel que précédemment définie, caractérisée en ce qu'elle est choisie dans le groupe constitué des formulations cosmétiques anhydres, des formulations cosmétiques de type émulsion eau dans huile (E/H), des formulations cosmétiques de type émulsion huile dans eau (H/E), et des formulations cosmétiques sous la forme d'émulsions multiples (notamment E/H, H/E ou H/E/H), ou encore des formulations cosmétiques étant des dispersions.

Formulation cosmétique selon l'invention tel que précédemment définie, caractérisée en ce qu'elle comprend en outre au moins un des constituants choisis dans le groupe comprenant les gélifiants ou structurants de phase grasse, en particulier les cires, les gommes, les copolymères oléfiniques ou encore les élastomères de silicone, les liants, notamment les savons d'acide gras, les gélifiants ou épaississants de phase aqueuse tels que les homo- ou copolymères acryliques ou sulfoniques, notamment à base d'AMPS, les agents filmogènes, tels que les dérivés de PVP, les latex acryliques ou les résines de silicone, les dispersants tels que les esters d'acide gras, les agents émulsionnant E/H, H/E ou E/Si, les actifs, les agents photo-protecteurs ou filtres UV organiques ou inorganiques, les charges, les pigments éventuellement traités hydrophobes et/ou lipophobes, les poudres de structure sphérique et/ou lamellaire, organiques ou inorganiques (telles que la silice, le talc, le mica...), les fibres d'origine naturelle ou synthétique, les colorants, les séquestrants, les ajusteurs de pH, les parfums, les conservateurs, et leurs mélanges.

Formulation cosmétique selon l'invention tel que précédemment définie, caractérisée en ce qu'elle est exempte de silicones cycliques (cyclomethicones), notamment de dérivés de cyclotétrasiloxane et de cyclopentasiloxane.

Formulation cosmétique selon l'invention tel que précédemment définie, caractérisée en ce qu'elle est sous une forme choisie dans le groupe comprenant les fluides, les gels, les crèmes, les pâtes, les mousses, les produits compacts pressés ou coulés et les produits solides, par exemple sous forme de bâton.

Formulation cosmétique selon l'invention tel que précédemment définie, caractérisée en ce qu'elle est utilisée en tant que formulation de soin ou d'hygiène du visage et/ou du corps, formulation hydratante, formulation anti-âge (anti-rides et/ou raffermissante), formulation dépigmentante, formulation pro-pigmentante, formulation autobronzante, formulation amincissante, formulation déodorante, formulation anti-transpirante, formulation de protection contre les UV, formulation nettoyante, formulation démaquillante, formulation démaquillante biphasique, formulation pour le bain, formulation capillaire (notamment un shampooing, un après-shampooing, un produit de coiffage, notamment de défrisage), formulation de coloration capillaire, formulation maquillante (notamment un fond de teint, un rouge à lèvres, un brillant à lèvres, une formulation pour la coloration des lèvres, un fard à joues ou à paupières, un mascara, un eye-liner ou encore un vernis à ongles).

Formulation cosmétique selon l'invention tel que précédemment définie, caractérisée en ce que l'au moins ledit au moins un alcane en C ≥ 11 est ramifié, et en ce que ladite formulation cosmétique est choisie dans le groupe des formulations cosmétiques de type émulsion eau dans huile (E/H), des formulations cosmétiques de type émulsion huile dans eau (H/E), et des formulations cosmétiques sous la forme d'émulsions multiples (notamment E/H, H/E ou H/E/H).

Formulation cosmétique selon l'invention tel que précédemment définie, caractérisée en ce que ledit au moins un alcane en C ≥ 11 est au moins un C12 ramifié, et en ce que ladite formulation cosmétique est choisie dans le groupe des formulations cosmétiques de type émulsion eau dans huile (E/H), des formulations cosmétiques de type émulsion huile dans eau (H/E), et des formulations cosmétiques sous la forme d'émulsions multiples (notamment E/H, H/E ou H/E/H).

Formulation cosmétique selon l'invention tel que précédemment définie, caractérisée en ce que ledit au moins un alcane en C ≥ 11 est au moins un C14 ramifié, et en ce que ladite formulation cosmétique est choisie dans le groupe des formulations cosmétiques de type émulsion eau dans huile (E/H), des formulations cosmétiques de type émulsion huile dans eau (H/E), et des formulations cosmétiques sous la forme d'émulsions multiples (notamment E/H, H/E ou H/E/H).

Formulation cosmétique selon l'invention tel que précédemment définie, caractérisée en ce que ledit au moins un alcane en C ≥ 11 est au moins un mélange de C12 ramifié et de C14 ramifié, et en ce que ladite formulation cosmétique est choisie dans le groupe des formulations cosmétiques de type émulsion eau dans huile (E/H), des formulations cosmétiques de type émulsion huile dans eau (H/E), et des formulations cosmétiques sous la forme d'émulsions multiples (notamment E/H, H/E ou H/E/H).

### EXEMPLES

### Partie A - exemples de compositions cosmétiques

### Exemple A1: émulsions / laits spray hors invention

Les formulations suivantes selon l'invention sont préparées selon les techniques bien connues de l'homme du métier comme par exemple les techniques décrites dans Conception des cosmétiques, La formulation, Anne Marie Pensé-Lhéritier, juillet 2014, Lavoisier :

| Formulation 1 (émulsion / lait spray) | | | |
|---|---|---|---|
| Phase | Nom commercial | INCI | Pourcentage massique (%) |
| A | Eau | Aqua | Qsp 100 |
| B | Isolan PDI (EVONIK) | Diisostearoyl Polyglyceryl-3 Dimer Dilinoleate | 2,5 |
| | Pongamia Butter 80 Plus (BIOSYNTHIS) | Pongamina glabra seed oil (and) C18-21 alkane | 10 |
| | UV Cut TiO2-60-VL (GRANT INDUSTRIES INC.) | Titanium dioxide (and) Coconut Alkanes (and) Stearic acid (and) Polyhdroxystearic acid (and) Alumina (and) Coco-caprylate/caprate | 20 |
| | Mélange de n-décane (30%) et de n-tetradecane (70%) | Tetradecane (and) Decane | 25 |
| C | MinaSolve Green C (MINASOLVE) | Pentylene Glycol (and) Glyceryl Caprylate/Caprate | 2 |

| Formulation 2 (émulsion / lait spray) | | | |
|---|---|---|---|
| Phase | Nom commercial | INCI | Pourcentage massique (%) |
| A | Eau | Aqua | Qsp 100 |
| B | Isolan PDI (EVONIK) | Diisostearoyl Polyglyceryl-3 Dimer Dilinoleate | 2,5 |
| | Pongamia Butter 80 Plus (BIOSYNTHIS) | Pongamina glabra seed oil (and) C18-21 alkane | 10 |
| | UV Cut TiO2-60-VL (GRANT INDUSTRIES INC.) | Titanium dioxide (and) Coconut Alkanes (and) Stearic acid (and) Polyhdroxystearic acid (and) Alumina (and) Coco-caprylate/caprate | 20 |
| | Mélange de n-décane (30%) et d'isohexadecane (70%) | Isohexadecane (and) Decane | 25 |
| C | MinaSolve Green C (MINASOLVE) | Pentylene Glycol (and) Glyceryl Caprylate/Caprate | 2 |

Les formulations 1 et 2 sont préparées comme suit :
- chauffage des phases A et B (60°C) ;
- émulsification de la phase A avec la phase B (agitation) ;
- ajout de la phase C tout en continuant l'agitation.

La stabilité est mesurée par la technique de l'analyse visuelle. Un échantillon de chacune des émulsions est placé dans un contenant transparent et observé à l'œil nu à intervalle de temps régulier, comme préconisé dans le rapport technique TR 13097 : Guidelines for the characterization of dispersion stability.

La formulation 2 est très stable (pas de déphasage après trois semaines), alors que la formulation 1 est déphasée au bout d'une journée.
Il est donc mis en évidence l'intérêt particulier de l'inclusion d'alcanes en C ≥ 11 ramifiés, dans le cadre de l'utilisation d'un excipient cosmétique selon l'invention dans une formulation de type émulsion.

### Exemple A2: mascara hors invention

Les formulations suivantes selon l'invention sont préparées :

| Formulation 3 (émulsion / mascara) | | | |
|---|---|---|---|
| Phase | Nom commercial | INCI | Pourcentage massique (%) |
| A | Eau | Aqua | 18,00 |
| | NaCl | Sodium Chloride | 0,70 |
| B | Natural Beewax | Cera Alba | 6,00 |
| | Sunflower Wax | Helianthus Annuus Seed Wax | 4,00 |
| | Mélange de n-décane (50%) et d'isohexadecane (50%) | Decane (and) Isohexadecane | Qsp 100,00 |
| | Xiameter^{™} RSN-0749 Resin (DOW CORNING) | Cyclopentasiloxane (and) Trimethylsiloxysilicate | 5,00 |
| | Nikkomulese WO (NIKKOL) | Cyclopentasiloxane (and) PEG-10 Dimethicone (and) Disteardimonium Hectorite | 16,00 |
| C | SA C335000-10 (MIYOSHI GROUP) | CI 77499, Dimethicone | 20,00 |
| | Micropearl M305 (SEPPIC) Methylmethacrylate crosspolymer 3,00 | Methylmethacrylate crosspolymer | 3,00 |

| Formulation 4 (émulsion / mascara) | | | |
|---|---|---|---|
| Phase | Nom commercial | INCI | Pourcentage massique (%) |
| A | Eau | Aqua | 18,00 |
| | NaCl | Sodium Chloride | 0,70 |
| B | Natural Beewax | Cera Alba | 6,00 |
| | Sunflower Wax | Helianthus Annuus Seed Wax | 4,00 |
| | Mélange de n-décane (50%) et de 5-methylundecane (50%) | Decane (and) Isohexadecane | Qsp 100,00 |
| | Xiameter^{™} RSN-0749 Resin (DOW CORNING) | Cyclopentasiloxane (and) Trimethylslloxysilicate | 5,00 |
| | Nikkomulese WO (NIKKOL) | Cyclopentasiloxane (and) PEG-10 Dimethicone (and) Disteardimonium Hectorite | 16,00 |
| C | SA C335000-10 (MIYOSHI GROUP) | CI 77499, Dimethicone | 20,00 |
| | Micropearl M305 (SEPPIC) Methylmethacrylate crosspolymer 3,00 | Methylmethacrylate crosspolymer | 3,00 |

Les formulations 3 et 4 sont préparées comme suit :
- préparation de la Phase A et chauffage (75°C) ;
- préparation de la Phase B et chauffage (85°C) ;
- mélange des Phases A et B ;
- ajout de la Phase C dans la Phase B sous agitation forte ;
- porter le mélange à 80°C, et ajout de la Phase A dans les Phases B+C ;
- mélange durant 15 minutes ;
- refroidissement à 25°C.

### Exemple A3 : formulation selon l'invention (formulation démaquillante biphasique)

La formulation suivante selon l'invention est préparée :

| Formulation 5 (formulation démaquillante biphasique) | | | |
|---|---|---|---|
| Phase | Nom commercial | INCI | Pourcentage massique (%) |
| A (phase huileuse) | Mélange de n-décane (30%), de n-dodecane (40%) et de n-tetradecane (30%) | Decane (and) | 99,60 |
| | | Dodecane (and) | |
| | | Tetradecane | |
| | Solution de CI 60725 à 0,5% dans SOUALANE | Squalane (and) CI 60725 | 0,20 |
| | Solution de CI 61565 à 0,5% dans SQUALANE | Squalane (and) CI 61565 | 0,20 |
| B (phase aqueuse) | Eau | Aqua | 85,70 |
| | K2HPO4 | Dipotassium Phosphate | 0,77 |
| | Acide citrique | Citric Acid | 0,48 |
| | Glycérine | Glycerine | 5,00 |
| | Chlorure de sodium | Sodium Chloride | 0,05 |
| | Hexylene glycol | Hexylene Glycol | 2,00 |
| | 1,3-propanediol | propanediol | 5,00 |
| | Solution aqueuse 0,5% CI 42090 | Aqua (and) CI 42090 | 1,00 |

Il est à noter que pour cette formulation 5, les pourcentages massiques sont donnés par rapport à la masse totale de la phase concernée.

Dans ladite formulation 5, les proportions phase aqueuse/phase huileuse sont de 50/50.

### Exemple A4: formulation pour la coloration-des lèvres, hors invention.

La formulation suivante selon l'invention est préparée :

| Formulation 6 (formulation pour la coloration des lèvres) | | | |
|---|---|---|---|
| Phase | Nom commercial | INCI | Pourcentage massique (%) |
| A | ViscoplastGreen 3000 (BIOSYNTHIS) | Dilinoleicacid/ Propanediolcopolymer | QSP 100,00 |
| | VegelightSilk et Polypropylsilsesquioxane | Decane (and) Tetradecane (and) Polypropylsilsesquioxane | 6,00 |
| | Biosynth DIM-18 | Diisostearylmalate | 5,00 |
| | Huile d'argan | Argania spinosa (Argan) seedoil | 3,00 |
| | Huile de Jojoba | Simmondsia chinensis oil | 3,00 |
| | Tinogard TT | Pentaerythrityl tetra-di-t-butylhydroxyhydrocinnamate | 0,05 |
| | Broyat DC Red 7 à 1% dans Biosynth DIM-18 | Diisostearylmalate (and) CI15850 | 25,00 |

### Exemple A5 : formulation selon l'invention (gloss à lèvre haute tenue)

| Formulation 7 (gloss à lèvre haute tenue) | | | |
|---|---|---|---|
| Phase | Nom commercial | INCI | Pourcentage massique (%) |
| | Cire de Candelilla | Candelilla cera | 6,16 |
| | Huile de Tournesol | Helianthus annuus seed oil | 5,00 |
| | Huile d'Amande douce | Prunus amygdalus dulcis oil | 4,00 |
| | Vegelight Silk | Coconut Alkanes | 50,00 |
| | 21% Decane | | |
| | 74% Dodecane | | |
| | 5% Tetradecane | | |
| A | Viscoplast Green 700 HVL | C18-21 alkane (and) Dilinoleic acid/Propanediol Copolymer (and) Coco-caprylate/caprate | 20,00 |
| | Mélange de SKYLINE F et de FDC YELLOW 6 AI-I2 (50% / 50%) | C10-18 TRIGLYCERIDES, POLYISOPRENE, ACETYLATED GLYCOL STEARATE, GLYCINE SOJA OIL, LECITHIN | 1,70 |
| | | CI 15985:1, SOPROPYL TITANIUM TRIISOSTEARATE | |
| | Mélange de SKYLINE F et de SA-TAO77891-10 (50% / 50%) | C10-18 TRIGLYCERIDES, POLYISOPRENE, ACETYLATED GLYCOL | 3,64 |
| | | STEARATE, GLYCINE SOJA OIL, LECITHIN | |
| | | CI 77891, DIMETHICONE | |
| | Mélange de SKYLINE F et de SA-C33128-10 (50% / 50%) | C10-18 TRIGLYCERIDES, POLYISOPRENE, ACETYLATED GLYCOL STEARATE, GLYCINE SOJA OIL, LECITHIN | 2,70 |
| | | CI 77491, DIMETHICONE | |
| | Mélange de SKYLINE F (60%) et de DC RED 27 AI-I2 (40%) | C10-18 TRIGLYCERIDES, POLYISOPRENE, ACETYLATED GLYCOL STEARATE, GLYCINE SOJA OIL, LECITHIN | 3,05 |
| | | CI 45410:2, SOPROPYL TITANIUM TRIISOSTEARATE | |
| | Mélange de SKYLINE F (60%) et de DC RED 7 Ca-C-TTB2 (40%) | C10-18 TRIGLYCERIDES, POLYISOPRENE, ACETYLATED GLYCOL STEARATE, GLYCINE SOJA OIL, LECITHIN | 3,75 |
| | | CI 15850:1, SOPROPYL TITANIUM TRIISOSTEARATE | |

### Exemple A6 : comparaison de formulations de sérum cheveux

Les formulations de sérum cheveux suivantes ont été comparées quant à leurs propriétés lors de l'application sur les cheveux.

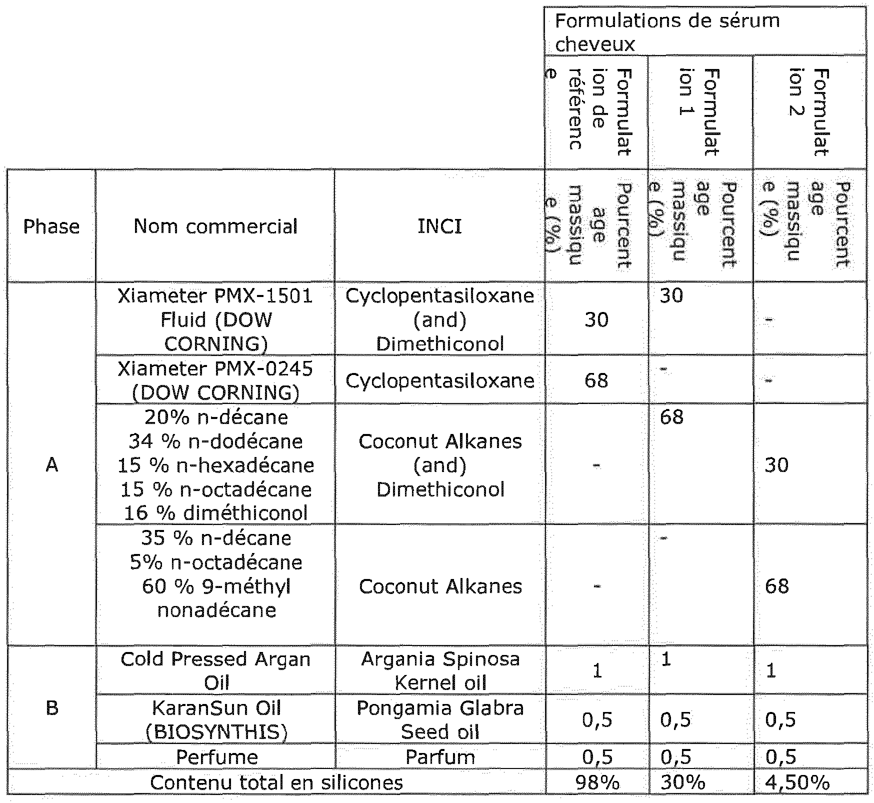

Les formulations de sérum cheveux ont été comparées comme suit :
3 mèches de cheveux d'environ 4 g et d'une longueur d'environ 17 cm sont lavées énergiquement avec 25 ml de solution de Texapon NSO à 12% et sont rincées puis séchées.
0,2 g des formulations décrites dans le tableau ci-dessus sont appliqué sur chacune des mèches afin de comparer lesdites formulations.
La mèche traitée avec la Formulation 2 présente des qualités meilleures que la Formulation de référence en particulier eu égard à la facilité de coiffage ; en revanche la mèche traitée avec la Formulation 1 n'est pas adaptée aux cheveux très fins.

### Exemple A7 : baume cheveux

### Baume référence

| **Nom commercial** | **Nom INCl** | **%** |
|---|---|---|
| Xiameter^{™} PMX-1501 Fluid | Cyclopentasiloxane (and) Dimethiconol | 25,00 |
| Belsil^{®} DM 60000 | Dimethicone | 3,00 |
| Marcol 82 | Paraffinum Liquidum | 25,00 |
| DOW CORNING^{®} 245 | Cyclopentasiloxane | 46,00 |
| Parfum | Perfume | 1,00 |

### Baume exemple A7a

| **Nom commercial** | **Nom INCl** | % |
|---|---|---|
| Xiameter^{™} PMX-1501 Fluid | Cyclopentasiloxane (and) Dimethiconol | 25,00 |
| Belsil^{®} DM 60000 | Dimethicone | 3,00 |
| Marcol 82 | Paraffinum Liquidum | 25,00 |
| *30% Decane* | Coconut alkanes | 46,00 |
| *40% Dodecane* | | |
| *30% Tetradecane* | | |
| Parfum | Perfume | 1,00 |

### Baume exemple A7b (hors invention)

| **Nom commercial** | **Nom INCI** | **%** |
|---|---|---|
| Xiameter^{™} PMX-1501 Fluid | Cyclopentasiloxane (and) Dimethiconol | 25,00 |
| Belsil^{®} DM 60000 | Dimethicone | 3,00 |
| Marcol 82 | Paraffinum Liquidum | 25,00 |
| *35% Decane* | Coconut alkanes | 46,00 |
| *5% Octadecane* | | |
| *60% 9-methyl Nonadecane* | | |
| Parfum | Perfume | 1,00 |

Les baumes A7a et A7b permettent d'obtenir des touchers sur la main huileux légers et soyeux sur le cheveux.

Les cheveux sont brillants et faciles à coiiffer.

### PARTIE B - Caractéristiques physicochimiques des excipients selon l'invention

### Exemple B1 : caractéristiques d'évaporation d'un excipient comprenant un mélange d'alcane selon l'invention

Un mélange d'alcane comprenant 30% de n-decane, 40% de n-dodecane et 30% de n-tetradecane a été investigués quant à ses caractéristiques d'évaporation.

Les conditions d'évaporation sont les suivantes : dessiccateur LABOMODERNE THERMOBALANCE KERN DBS 60-3, 20°C, sous atmosphère contrôlée.

La courbe d'évaporation obtenue est donnée ci-après :
Les mesures des taux d'évaporation du mélange comprenant 30% de n-decane, 40% de n-dodécane et 30% de n-tetradécane sont données ci-après en comparaison avec celles d'un mélange selon l'art antérieur comprenant 70% de n-dodécane, et 30% de n-tetradécane et celles du cyclopentasiloxane.

| Evaporation d'un excipient selon l'invention (n-décane (30%), n-dodecane (40%) et n-tétradécane (30%)) | | |
|---|---|---|
| Temps (min) | Masse totale (g) | Masse résiduelle (%) |
| 0 | 5,32 | 100 |
| 7 | 5,31 | 99,68 |
| 20 | 5,30 | 99,23 |
| 39 | 5,28 | 98,61 |
| 59 | 5,27 | 98,01 |
| 79 | 5,25 | 97,36 |
| 104 | 5,23 | 96,70 |
| 129 | 5,21 | 96,05 |
| 159 | 5,19 | 95,33 |
| 189 | 5,17 | 94,62 |
| 219 | 5,15 | 94,04 |
| 249 | 5,13 | 93,28 |
| 279 | 5,10 | 92,55 |
| 309 | 5,09 | 92,00 |
| 358 | 5,05 | 90,54 |
| 429 | 5,01 | 89,39 |
| 479 | 4,98 | 88,23 |
| 1438 | 4,42 | 69,27 |

| Evaporation d'un mélange d'alcane comparatif (n-dodecane (70%) et n-tétradécane (30%)) | | |
|---|---|---|
| Temps (min) | Masse totale (g) | Masse résiduelle (%) |
| 0 | 5,38 | 100,00 |
| 20 | 5,38 | 99,83 |
| 50 | 5,37 | 99,51 |
| 90 | 5,36 | 99,13 |
| 130 | 5,34 | 98,78 |
| 170 | 5,33 | 98,40 |
| 210 | 5,32 | 98,00 |
| 250 | 5,31 | 97,61 |
| 300 | 5,30 | 97,21 |
| 356 | 5,28 | 96,65 |
| 1620 | 5,00 | 87,14 |

| Evaporation du Cyclopentasiloxane (D5) | | |
|---|---|---|
| Temps (min) | Masse totale (g) | Masse résiduelle (%) |
| 0 | 5,39 | 100 |
| 7 | 5,38 | 99,82 |
| 20 | 5,37 | 99,55 |
| 39 | 5,36 | 99,16 |
| 59 | 5,35 | 98,75 |
| 79 | 5,33 | 98,31 |
| 104 | 5,32 | 97,82 |
| 129 | 5,31 | 97,36 |
| 159 | 5,29 | 96,85 |
| 189 | 5,28 | 96,44 |
| 219 | 5,26 | 95,94 |
| 249 | 5,25 | 95,51 |
| 279 | 5,24 | 95,12 |
| 309 | 5,23 | 94,55 |
| 356 | 5,20 | 93,72 |
| 429 | 5,17 | 92,74 |
| 479 | 5,14 | 91,97 |
| 1436 | 4,54 | 71,85 |

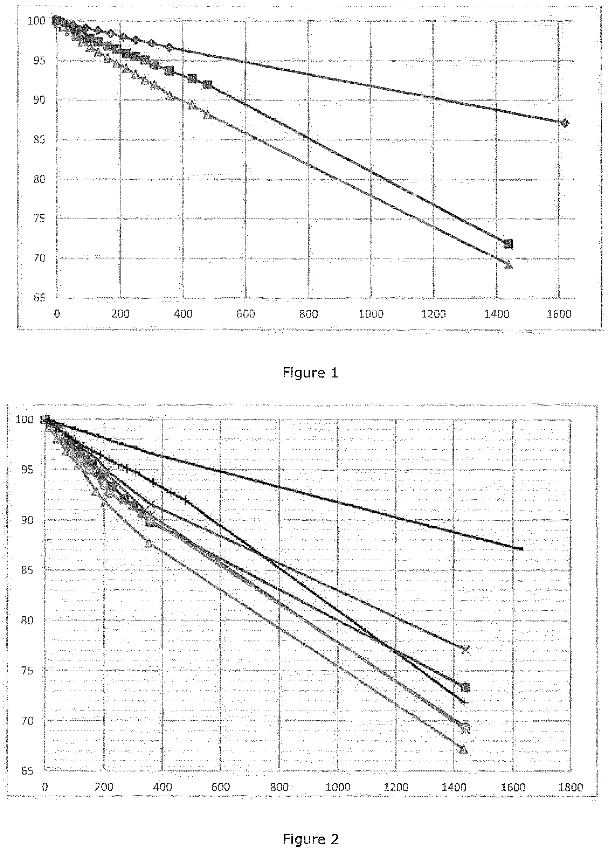

Les résultats sont représentés sur la Figure 1, la courbe représentée par des losanges est la courbe d'évaporation d'un mélange selon l'art antérieur comprenant 70% de n-dodécane, et 30% de n-tetradécane, la courbe représentée par les carrés est la courbe d'évaporation du cyclopentasiloxane et la courbe représentée par les losanges est la courbe d'évaporation d'un mélange selon l'invention comprenant 30% de n-decane, 40% de n-dodécane et 30% de n-tetradécane.

On observe que la courbe du mélange selon l'invention est parfaitement alignée avec celle du cyclopentasiloxane.

De plus, il a été mis en évidence que l'évaporation est particulièrement surprenante et non déductible de celle de chacun des alcanes pris séparément.

### Exemple B2 : caractéristiques d'évaporation d'un excipient comprenant un mélange d'alcane selon l'invention

Plusieurs autres excipients selon l'invention (mélanges d'alcanes) ont été testés quant à leur vitesse d'évaporation, dans des conditions identiques à celles précédemment décrites.

### Mélanges n-décane / n-dodécane / n-tétradécane

Un mélange d'alcanes constitué de 10% de n-décane, 85% de n-dodécane et 5% de n-tétradécane a été testé. Les résultats sont présentés dans le tableau ci-dessous :

| Evaporation d'un excipient selon l'invention (10% de n-décane, 85% de n-dodécane et 5% de n-tétradécane) | | |
|---|---|---|
| Temps (min) | Masse totale (g) | Masse résiduelle (%) |
| 0 | 5,52 | 100 |
| 20 | 5,51 | 99,53 |
| 50 | 5,49 | 98,98 |
| 90 | 5,46 | 98,03 |
| 120 | 5,44 | 97,29 |
| 180 | 5,39 | 95,98 |
| 210 | 5,36 | 94,90 |
| 360 | 5,26 | 91,58 |
| 1440 | 4,81 | 77,10 |

Un mélange d'alcanes constitué de 15% de n-décane, 80% de n-dodécane et 5% de tétradécane a été testé. Les résultats sont présentés dans le tableau ci-dessous :

| Evaporation d'un excipient selon l'invention (15% de n-décane, 80% de n-dodécane et 5% de tétradécane) | | |
|---|---|---|
| Temps (min) | Masse totale (g) | Masse résiduelle (%) |
| 0 | 5,46 | 100 |
| 20 | 5,44 | 99,43 |
| 50 | 5,42 | 98,74 |
| 90 | 5,39 | 97,58 |
| 120 | 5,36 | 96,63 |
| 180 | 5,31 | 95,02 |
| 210 | 5,29 | 94,52 |
| 360 | 5,17 | 90,51 |
| 1440 | 4,52 | 69,14 |

Un mélange d'alcanes constitué de 20% de n-décane, 75,6% de n-dodécane et 4,4% de tétradécane a été testé. Les résultats sont présentés dans le tableau ci-dessous :

| Evaporation d'un excipient selon l'invention (20% de n-décane, 75,6% de n-dodécane et 4,4% de tétradécane) | | |
|---|---|---|
| Temps (min) | Masse totale (g) | Masse résiduelle (%) |
| 0 | 5,43 | 100 |
| 30 | 5,40 | 99,04 |
| 50 | 5,38 | 98,33 |
| 90 | 5,33 | 96,66 |
| 120 | 5,31 | 95,87 |
| 150 | 5,28 | 94,95 |
| 200 | 5,23 | 93,40 |
| 220 | 5,21 | 92,63 |
| 360 | 5,13 | 89,99 |
| 1440 | 4,51 | 69,42 |

Un mélange d'alcanes constitué de 20% de n-décane, 74% de n-dodécane et 6% de tétradécane a été testé. Les résultats sont présentés dans le tableau ci-dessous :

| Evaporation d'un excipient selon l'invention (20% de n-décane, 74% de n-dodécane et 6% de tétradécane) | | |
|---|---|---|
| Temps (min) | Masse totale (g) | Masse résiduelle (%) |
| 0 | 5,37 | 100 |
| 20 | 5,36 | 99,49 |
| 40 | 5,34 | 98,93 |
| 60 | 5,32 | 98,25 |
| 80 | 5,31 | 97,79 |
| 100 | 5,29 | 97,23 |
| 120 | 5,27 | 96,64 |
| 140 | 5,25 | 95,96 |
| 160 | 5,24 | 95,44 |
| 190 | 5,21 | 94,39 |
| 230 | 5,17 | 93,24 |
| 270 | 5,14 | 92,06 |
| 300 | 5,12 | 91,43 |
| 330 | 5,09 | 90,58 |
| 360 | 5,07 | 89,72 |
| 1440 | 4,57 | 73,36 |

Un mélange d'alcanes constitué de 20% de n-décane, 70% de n-dodécane et 10% de tétradécane a été testé. Les résultats sont présentés dans le tableau ci-dessous :

| Evaporation d'un excipient selon l'invention (20% de n-décane, 70% de n-dodécane et 10% de tétradécane) | | |
|---|---|---|
| Temps (min) | Masse totale (g) | Masse résiduelle (%) |
| 0 | 5,38 | 100 |
| 20 | 5,36 | 99,51 |
| 40 | 5,35 | 98,94 |
| 60 | 5,33 | 98,29 |
| 80 | 5,31 | 97,83 |
| 100 | 5,30 | 97,25 |
| 120 | 5,28 | 96,68 |
| 140 | 5,26 | 96,02 |
| 160 | 5,28 | 95,50 |
| 190 | 5,21 | 94,46 |
| 230 | 5,18 | 93,31 |
| 270 | 5,14 | 92,11 |
| 300 | 5,12 | 91,50 |
| 330 | 5,10 | 90,67 |
| 360 | 5,07 | 89,81 |
| 1440 | 4,58 | 73,31 |

Un mélange d'alcanes constitué de 20% de n-décane, 75% de n-dodécane et 5% de tétradécane a été testé. Les résultats sont présentés dans le tableau ci-dessous :

| Evaporation d'un excipient selon l'invention (20% de n-décane, 75% de n-dodécane et 5% de tétradécane) | | |
|---|---|---|
| Temps (min) | Masse totale (g) | Masse résiduelle (%) |
| 0 | 5,45 | 100 |
| 13 | 5,43 | 99,17 |
| 43 | 5,39 | 98,07 |
| 73 | 5,36 | 96,78 |
| 113 | 5,32 | 95,45 |
| 173 | 5,24 | 92,86 |
| 203 | 5,20 | 91,77 |
| 353 | 5,08 | 87,70 |
| 1433 | 4,46 | 67,23 |

La Figure 2 représente le pourcentage de matière non évaporée (exprimé en %) en fonction du temps (exprimé en secondes) d'un certain nombre de composé (cyclopentasiloxane) et mélanges d'alcanes.

La courbe représentée par des « - » (la courbe qui présente le mélange ayant le moins tendance à s'évaporer du graphique) est la courbe d'évaporation d'un mélange d'alcanes selon l'art antérieur comprenant 70% de n-dodécane et 30% de n-tétradécane.

La courbe représentée par des « + » est la courbe d'évaporation du cyclopentasiloxane.

La courbe représentée par des losanges est la courbe d'évaporation d'un mélange selon l'invention comprenant 20% de n-décane, 74% de n-dodécane et 6% de n-tétradécane.

La courbe représentée par des carrés est la courbe d'évaporation d'un mélange selon l'invention comprenant 20% de n-décane, 70% de n-dodécane et 10% de n-tétradécane.

La courbe représentée par des triangles est la courbe d'évaporation d'un mélange selon l'invention comprenant 20% de n-décane, 75% de n-dodécane et 5% de n-tétradécane.

La courbe représentée par des « x » est la courbe d'évaporation d'un mélange selon l'invention comprenant 10% de n-décane, 85% de n-dodécane et 5% de n-tétradécane.

La courbe représentée par les étoiles est la courbe d'évaporation d'un mélange selon l'invention comprenant 15% de n-décane, 80% de n-dodécane et 5% de n-tétradécane.

La courbe représentée par les ronds est la courbe d'évaporation d'un mélange selon l'invention comprenant 20% de n-décane, 70% de n-dodécane et 10% de n-tétradécane.

On remarque que les mélanges selon l'invention ont un profil d'évaporation plus proche du cyclopentasiloxane que le mélange d'alcanes selon l'art antérieur.

### Mélange n-décane / isohexadécane (hors invention)

Un mélange d'alcanes constitué de 30% de n-décane et 70% d'isohexadécane a été testé. Les résultats sont présentés dans le tableau ci-dessous :

| Evaporation d'un excipient selon l'invention (30% de n-décane et 70% d'isohexadécane) | | |
|---|---|---|
| Temps (min) | Masse totale (g) | Masse résiduelle (%) |
| 0 | 5,41 | 100 |
| 20 | 5,38 | 99,15 |
| 50 | 5,33 | 97,46 |
| 90 | 5,26 | 95,15 |
| 130 | 5,20 | 93,13 |
| 170 | 5,14 | 91,07 |
| 210 | 5,07 | 88,86 |
| 250 | 5,01 | 86,92 |
| 300 | 4,96 | 85,05 |
| 360 | 4,89 | 82,74 |
| 1620 | 4,27 | 62,38 |

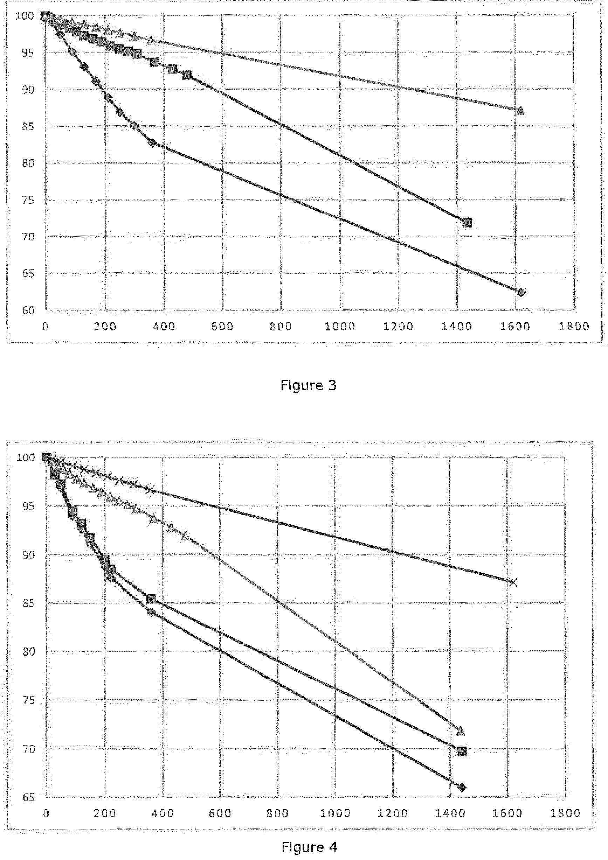

La Figure 3 représente le pourcentage de matière non évaporée (exprimé en %) en fonction du temps (exprimé en secondes) d'un certain nombre de composé (cyclopentasiloxane) et mélanges d'alcanes.

La courbe représentée par des triangles (la courbe qui présente le mélange ayant le moins tendance à s'évaporer du graphique) est la courbe d'évaporation d'un mélange d'alcanes selon l'art antérieur comprenant 70% de n-dodécane et 30% de n-tétradécane.

La courbe représentée par les carrés est la courbe d'évaporation du cyclopentasiloxane.

La courbe représentée par les losanges est la courbe d'évaporation d'un mélange selon l'invention comprenant 30% de n-décane et 70% d'isohexadécane.

On remarque que les mélanges selon l'invention ont un profil d'évaporation plus proche du cyclopentasiloxane que le mélange d'alcanes selon l'art antérieur, à partir d'environ T = 900 secondes.

Il est par ailleurs notable qu'en dépit de son grand nombre de carbone (C16), l'isohexadecane permet d'obtenir un excipient très volatile (par exemple, plus volatile que la même formulation comprenant, à la place de l'isohexadécane, un mélange de n-dodécane et de n-tétradécane, voir exemples présentés ci-dessus).

Des mesures de viscosité à 40°C ont été effectuées sur les composés ou mélange suivants :
- le cyclopentasiloxane : 3,04 mm²/s ;
- l'isohexadécane : 3,18 mm²/s ; et
- le mélange n-décane (30%) / isohexadécane (70%) : 1,89 mm²/s.

Il est notable que l'utilisation de l'isohexadécane permet d'obtenir une viscosité proche de celle du cyclopentasiloxane.

### Mélanges n-décane / isododécane / isoeicosane (hors invention)

Un mélange d'alcanes constitué de 5% de n-décane, 30% d'isododécane et 65% d'isoeicosane (C20) a été testé. Les résultats sont présentés dans le tableau ci-dessous :

| Evaporation d'un excipient selon l'invention (5% de n-décane, 30% d'isododécane et 65% d'isoeicosane) | | |
|---|---|---|
| Temps (min) | Masse totale (g) | Masse résiduelle (%) |
| 0 | 5,44 | 100 |
| 30 | 5,38 | 98,13 |
| 50 | 5,35 | 96,90 |
| 90 | 5,26 | 93,94 |
| 120 | 5,22 | 92,64 |
| 150 | 5,17 | 91,16 |
| 200 | 5,10 | 88,76 |
| 220 | 5,06 | 87,57 |
| 360 | 4,96 | 84,07 |
| 1440 | 4,41 | 66,02 |

Un mélange d'alcanes constitué de 5% de n-décane, 25% d'isododécane et 70% d'isoeicosane (C20) a été testé. Les résultats sont présentés dans le tableau ci-dessous :

| Evaporation d'un excipient selon l'invention (5% de n-décane, 25% d'isododécane et 70% d'isoeicosane) | | |
|---|---|---|
| Temps (min) | Masse totale (g) | Masse résiduelle (%) |
| 0 | 5,42 | 100 |
| 30 | 5,37 | 98,27 |
| 50 | 5,34 | 97,27 |
| 90 | 5,25 | 94,47 |
| 120 | 5,21 | 93,22 |
| 150 | 5,17 | 91,75 |
| 200 | 5,10 | 89,48 |
| 220 | 5,07 | 88,47 |
| 360 | 4,98 | 85,43 |
| 1440 | 4,50 | 69,76 |

La Figure 4 représente le pourcentage de matière non évaporée (exprimé en %) en fonction du temps (exprimé en secondes) d'un certain nombre de composé (cyclopentasiloxane) et mélanges d'alcanes.

Sur la figure 4, la courbe représentée par les « x » est la courbe d'évaporation d'un mélange selon l'art antérieur comprenant 70% de n-dodécane et 30% de n-tétradécane.

La courbe représentée par les triangles est la courbe d'évaporation du cyclopentasiloxane.

La courbe représentée par les losanges est la courbe d'évaporation d'un mélange selon l'invention comprenant 5% de n-décane, 30% d'isododécane et 65% d'isoeicosane.

La courbe représentée par les carrés est la courbe d'évaporation d'un mélange selon l'invention comprenant 5% de n-décane, 25% d'isododécane et 70% d'isoeicosane.

On remarque que les mélanges selon l'invention ont un profil d'évaporation plus proche du cyclopentasiloxane que le mélange d'alcanes selon l'art antérieur, à partir d'environ T = 800 secondes.

### PARTIE C - Synthèse des alcanes constituant les excipients selon l'invention

### Exemple C1 - Synthèse du 2, 3, 6 triméthylheptane

### Synthèse de Guerbet d'un alcane en C10 à partir de l'alcool isoamylique

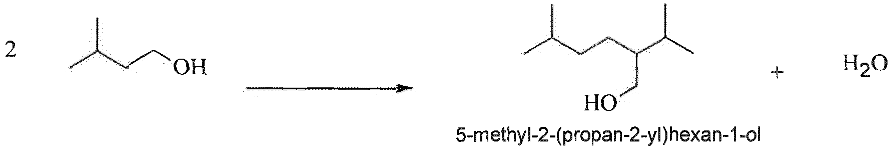

La réaction est réalisée dans les conditions suivantes :

### Réactifs utilisés

| | |
|---|---|
| Isoamyl alcool 99% | |
| m, g | 75,8 |
| n, mol | 0,86 |
| KOH 85% | |
| m, g | 1,1 |
| % (/alcool) | 1,5 |
| Catalyseur | |
| Nom | Pricat Cu 50/8 |
| m, mg | 19,0 |
| % (/alcool) | 0,025 |

### Conditions opératoires

| Paramètres fixés | |
|---|---|
| Température, °C | 180 à 230 |
| Durée, h | 24 |
| Bullage azote | Non |

### Bilan analytique de la synthèse de l'alcool de Guerbet C10

| Produit brut | |
|---|---|
| Composition (CPG) (voir méthode en annexe) | |
| Isoamyl alcool, % | <5% |
| Alcool iC10,% | >75% |
| Lourds, % | Max 20% |

Des produits plus lourds de type trimer C15 ont été obtenus comme impuretés. Une purification par distillation sous vide est donc nécessaire.

Un étêtage permet l'élimination des traces d'alcool isoamylique.

Un équeutage permet ensuite l'élimination des alcools plus lourds.

### Bilan analytique de la purification de l'alcool de Guerbet C10

| Produit fini | |
|---|---|
| Composition (CPG) (voir méthode en annexe) | |
| Isoamyl alcool, % | <1% |
| Alcool iC10,% | > 94% |
| Lourds, % | <5% |

### Déshydratation de l'alcool de Guerbet C10

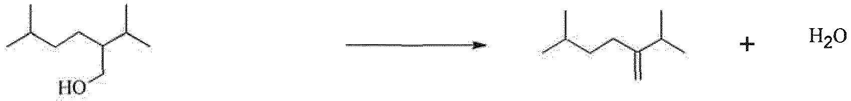

### 5-methyl-2-(propan-2-yl)hexan-1-ol

### Composition de l'alcool de Guerbet

| Matière première | Teneur, % | Fournisseur |
|---|---|---|
| 5-methyl-2-(propan-2-yl) hexan-1-ol | Min 94% | BIOSYNTHIS |

### Matières premières utilisées :

| **Matière première** | **Fournisseur** |
|---|---|
| Alumine | Johnson & Matthey |
| Laine de silice | VWR |
| Azote | Air Liquide |

### Déshydratation

Un lit de catalyseur est placé à la mi-hauteur du réacteur. Un thermocouple est placé au cœur de ce lit. Ce dernier est maintenu par une grille sur laquelle est placée de la laine de silice. Au dessus du lit, de la laine de silice est ajoutée afin de bloquer le lit de catalyseur.

Le catalyseur utilisé est une alumine industrielle fournie par Johnson & Matthey.

Le LHSV (Liquid Hourly Space Velocity) correspond au débit d'alcool passé au travers du réacteur exprimé en mL/min/mL de catalyseur.

### Résultats déshydratation

| Alcool | T, °C | LHSV | d (mL/min) | Conversion | Quantité d'eau produite pour 1 Tonne d'alcool, kg | Quantité d'alcene produite pour 1 Tonne d'alcool, kg |
|---|---|---|---|---|---|---|
| Alcool de guerbet C10 | 330 | 2,5 | 0,5 | >99% | 114 | 886 |

### Caractérisation alcènes

| Alcene | Formule Alcene | Indice d'iode alcene g I2/100g | Aspect à température ambiante |
|---|---|---|---|
| alcene ic10, | 94% C10H20 | 161 | Liquide |

### Hydrogénation des alcènes

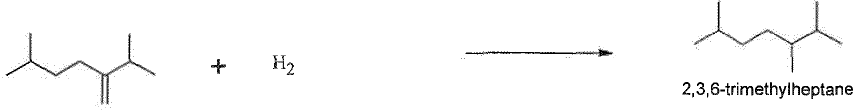

La réaction d'hydrogénation se déroule dans un réacteur batch d'un litre de capacité. Les conditions opératoires sont détaillées ci-dessous.

L'alcène 600g et le catalyseur (nickel de Raney) 3g, sont introduits dans le réacteur à température ambiante. Le réacteur est inerté par 3X5bars d'azote et la température est élevée à la température de travail de 180°C. 5bars d'hydrogène sont ensuite introduits. Au bout de deux heures la température est élevée à 200°C et la pression à 10bar. la réaction est ensuite maintenue 3h supplémentaires.

### Caractérisation de l'alcane

| Alcane | Consommation d'hydrogène pour 1 Tonne d'alcène, kq H2 | Formule alcane | Indice d'iode alcene g I2/100g | Aspect à température ambiante |
|---|---|---|---|---|
| Alcane ic10 | 12,7 | C10H22 | <1 | Liquide |

### Exemple C2 - Synthèse du 5-méthylundécane

### Synthèse de Guerbet C12 à partir de l'alcool hexanolique vegetal

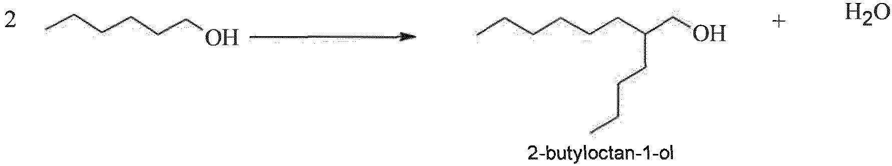

La réaction est réalisée dans les conditions suivantes

### Réactifs utilisés pour la synthèse du Guerbet C12

| | | |
|---|---|---|
| n-hexanol 99% | | |
| | m, g | 87,7 |
| | n, mol | 0,86 |
| KOH 85% | | |
| | m, g | 1,3 |
| | % (/alcool) | 1,5 |
| Catalyseur | | |
| | Nom | **Pricat Cu 50/8** |
| | m, mg | 22,0 |
| | % (/alcool) | 0,025 |

### Conditions opératoires de la synthèse Guerbet C12

| | |
|---|---|
| Température, °C | 180 à 230 |
| Durée, h | 24 |
| Bullaqe azote | Non |

### Bilan analytique de la synthèse de l'alcool de Guerbet C12

| | |
|---|---|
| Produit brut | |
| Composition (CPG) (voir méthode en annexe) | |
| hexanol alcool, % | <5% |
| 2 butyl octanol,% | >75% |
| Lourds, % | Max 20% |

Des produits plus lourds de type trimer C18 ont été obtenus comme sous-produits. Une purification par distillation sous vide est donc necessaire.

Un étêtage permet l'élimination des traces d'alcool hexanolique.

Un équeutage permet ensuite l'élimination des alcools plus lourds.

### Bilan analytique de la purification de l'alcool de Guerbet C12

| | |
|---|---|
| Produit fini | |
| Composition (CPG) (voir méthode en annexe) | |
| Isoamyl alcool, % | <1% |
| 2 butyl octanol,% | >94% |
| Lourds, % | <5% |

### Déshydratation de l'alcool de guerbet C12

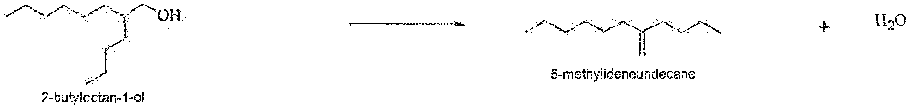

Les matières premières utilisées pour cette étude sont présentées dans le tableau suivant. Aucun réactif ou solvant dangereux n'est utilisé. Le catalyseur est une alumine industrielle.

### Composition de l'alcool de guerbet

| **Matières premières** | **Teneur, %** | **Fournisseurs** |
|---|---|---|
| 2 butyl octanol | Min 94% | BIOSYNTHIS |

### Matières premières utilisées :

| **Matières premières** | **Fournisseurs** |
|---|---|
| Alumine | Johnson & Matthey |
| Laine de silice | VWR |
| Azote | Air Liquide |

### Déshydratation

La déshydratation est mise en oeuvre selon le mode opératoire ci-dessus décrit.
Le LHSV (Liquid Hourly Space Velocity) correspond au débit d'alcool passé au travers du réacteur exprimé en mL/min/mL de catalyseur.

### Résultats déshydratation

| Alcool | T, °C | LHSV | d (mL/min) | Conversion | Quantité d'eau produite pour 1 Tonne d'alcool, kg | Quantité d'alcene produite pour 1 Tonne d'alcool, kg |
|---|---|---|---|---|---|---|
| Alcool de guerbet C12 | 330 | 2,5 | 0,5 | >99% | 96,7 | 903 |

### Caractérisation alcènes

| Alcene | Formule Alcene | Indice d'iode alcene g I2/100g | Aspect à température ambiante |
|---|---|---|---|
| alcene ic12, | 94% C12H24 | 151 | Liquide |

### Réaction d'hydrogénation

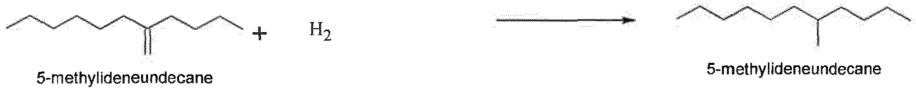

L'hydrogénation est mise en oeuvre selon le mode opératoire ci-dessus décrit.

### Caractérisation de l'alcane

| Alcane | Consommation d'hydrogène pour 1 Tonne d'alcène, kg H2 | Formule alcane | Indice d'iode alcene g I2/100g | Aspect à température ambiante |
|---|---|---|---|---|
| Alcane ic12 | 12,7 | C12H26 | < 1 | Liquide |

## Revendications

1. Excipient cosmétique comprenant un mélange d'alcanes, ledit mélange d'alcanes comprenant au moins un alcane en C8-C10, et au moins un alcane en C ≥ 11, **caractérisé en ce que** l'au moins un alcane en C8-C10 est un C10 et l'au moins un alcane en C≥11 est un alcane linéaire en C12 et **en ce que** ledit au moins un alcane en C8-C10 est présent en un pourcentage massique compris entre 10 et 30% (10 ≤ pourcentage massique ≤ 30 %), par rapport à la masse totale dudit mélange d'alcanes et **en ce que** ledit mélange d'alcanes est exempt d'alcanes dont le nombre de carbone est impair et **en ce que** ledit mélange d'alcane a un point éclair inférieur ou égal à 69°C (≤ 69°C) mesuré selon la norme ASTM D93.

2. Excipient cosmétique selon la revendication 1, **caractérisé en ce qu'**il est intégralement obtenu à partir de matières premières d'origine végétale, bactérienne ou animale.

3. Excipient cosmétique selon l'une quelconque des revendications précédentes, **caractérisé en ce qu'**il est intégralement obtenu à partir de matières premières d'origine végétale.

4. Excipient cosmétique comprenant un mélange d'alcanes selon l'une quelconque des revendications précédentes, **caractérisé en ce que** ledit au moins un alcane en C8-C10 est au moins un alcane linéaire ou ramifié en C10.

5. Excipient cosmétique comprenant un mélange d'alcanes selon l'une quelconque des revendications précédentes, **caractérisé en ce que** ledit au moins un alcane en C8-C10 est choisi dans le groupe constitué du 4-methylnonane (C₁₀H₂₂, CAS 17301-949), du 2-methylnonane (C₁₀H₂₂, CAS 871-83-0), du n-décane (C₁₀H₂₂, CAS 124-185), et de leurs mélanges.

6. Excipient cosmétique comprenant un mélange d'alcanes selon l'une quelconque des revendications précédentes, **caractérisé en ce que** ledit au moins un alcane en C8-C10 est au moins un méthyl alcane en C10.

7. Excipient cosmétique comprenant un mélange d'alcanes selon l'une quelconque des revendications précédentes, **caractérisé en ce que** ledit excipient comprend en outre du Polysilicone-11.

8. Excipient cosmétique selon l'une quelconque des revendications précédentes, **caractérisé en ce que** le pourcentage d'évaporation à t=10 heures est compris entre 10 et 30 (10% ≤ pourcentage d'évaporation à t=10 ≤ 30%).

## Patentansprüche

1. Kosmetisches Bindemittel umfassend eine Alkanmischung, wobei die Alkanmischung wenigstens ein C₈-C₁₀-Alkan und wenigstens ein Alkan mit C ≥ 11 umfasst, **dadurch gekennzeichnet, dass** das wenigstens eine C₈-C₁₀-Alkan ein C₁₀-Alkan und das wenigstens eine Alkan mit C ≥ 11 ein lineares C₁₂-Alkan ist und wobei das wenigstens eine C₈-C₁₀-Alkan mit einem Massenanteill zwischen 10 und 30 % (10 ≤ Massenanteil ≤ 30 %) bezogen auf die Gesamtmasse der Alkanmischung vorhanden ist und die Alkanmischung frei von Alkanen mit ungerader Kohlenstoff-Anzahl ist und wobei die Alkanmischung einen Flammpunkt von 69 °C oder weniger (≤ 69 °C) aufweist.

2. Kosmetisches Bindemittel nach Anspruch 1, **dadurch gekennzeichnet, dass** es vollständig erhalten ist aus Rohstoffen pflanzlichen, bakteriellen oder tierischen Ursprungs.

3. Kosmetisches Bindemittel nach einem der vorangehenden Ansprüchen, **dadurch gekennzeichnet, dass** es vollständig erhalten ist aus Rohstoffen pflanzlichen Ursprungs.

4. Kosmetisches Bindemittel umfassend eine Alkanmischung nach einem der vorangehenden Ansprüche, **dadurch gekennzeichnet, dass** das wenigstens eine C₈-C₁₀-Alkan wenigstens ein lineares oder ein verzweigtes C₁₀-Alkan ist.

5. Kosmetisches Bindemittel umfassend eine Alkanmischung nach einem der vorangehenden Ansprüche, **dadurch gekennzeichnet, dass** das wenigstens eine C₈-C₁₀-Alkan ausgewählt ist aus der Gruppe bestehend aus 4-Methylnonan (C₁₀H₂₂, CAS 17301-949), 2-Methylnonan (C₁₀H₂₂, CAS 871-83-0), n-Dekan (C₁₀H₂₂, CAS 124-185) und Mischungen davon.

6. Kosmetisches Bindemittel umfassend eine Alkanmischung nach einem der vorangehenden Ansprüche, **dadurch gekennzeichnet, dass** das wenigstens eine C₈-C₁₀-Alkan wenigstens ein C₁₀-Methylalkan ist.

7. Kosmetisches Bindemittel umfassend eine Alkanmischung nach einem der vorangehenden Ansprüche, **dadurch gekennzeichnet, dass** das Bindemittel außerdem Polysilicon-11 umfasst.

8. Kosmetisches Bindemittel nach einem der vorangehenden Ansprüche, **dadurch gekennzeichnet, dass** der Verdunstungsprozentsatz bei t = 10 Stunden zwischen 10 und 30 (10 % ≤ Verdunstungsprozentsatz bei t = 10 ≤ 30 %) beträgt.

## Claims

1. A cosmetic excipient comprising a mixture of alkanes, said mixture of alkanes comprising at least one C₈-C₁₀ alkane and at least one C ≥ 11 alkane, **characterized in that** the at least one C₈-C₁₀ is a C₁₀ and the at least one C ≥ 11 alkane is a linear C₁₂ alkane, wherein said at least one C₈-C₁₀ alkane is present in a mass percentage of between 10 and 30% (10 ≤ percent by weight ≤ 30%) relative to the total mass of said mixture of alkanes, wherein said mixture of alkanes is free of alkanes with an odd carbon number, and wherein said mixture of alkanes has a flash point of less than or equal to 69°C (≤ 69 °C).

2. The cosmetic excipient according to claim 1, **characterized in that** it is obtained entirely from raw materials of plant origin, bacterial origin, or animal origin.

3. The cosmetic excipient according to any one of the preceding claims, **characterized in that** it is obtained entirely from raw materials of plant origin.

4. The cosmetic excipient comprising a mixture of alkanes according to any one of the preceding claims, **characterized in that** the at least one C₈-C₁₀ alkane is at least a linear or branched C₁₀ alkane.

5. The cosmetic excipient comprising a mixture of alkanes according to any one of the preceding claims, **characterized in that** the at least one C₈-C₁₀ alkane is selected from the group consisting of 4-methylnonane (C₁₀H₂₂, CAS 17301-94-9), 2-methylnonane (C₁₀H₂₂, CAS 871-83-0), n-decane (C₁₀H₂₂, CAS 124-18-5), and mixtures thereof.

6. The cosmetic excipient comprising a mixture of alkanes according to any one of the preceding claims, **characterized in that** the at least one C₈-C₁₀ alkane is at least a C₁₀ methyl alkane.

7. The cosmetic excipient comprising a mixture of alkanes according to any one of the preceding claims, **characterized in that** the excipient further comprises polysilicone-11.

8. The cosmetic excipient according to one of the preceding claims, **characterized in that** the percentage of evaporation at t=10 hours is between 10 and 30 (10% ≤ percentage of evaporation at t=10 ≤ 30%).
